# EUROPÄISCHE PATENTSCHRIFT

(11) **EP 1 109 766 B1**
(45) Veröffentlichungstag und Bekanntmachung des Hinweises auf die Patenterteilung: **03.11.2004**
(21) Anmeldenummer: 99968662.9
(22) Anmeldetag: 24.08.1999
(51) Int. Cl.: C07C 17/38, C07C 17/389, C07C 17/395, C07C 19/08, C01B 7/07, C01B 7/19

(54) **REINIGUNG VON 1,1,1,3,3-PENTAFLUORBUTAN**
PURIFICATION OF 1,1,1,3,3-PENTAFLUOROBUTANE
PURIFICATION DE 1,1,1,3,3-PENTAFLUOROBUTANE

(30) Priorität: 03.09.1998 DE 19840099
(43) Veröffentlichungstag der Anmeldung: 27.06.2001
(73) Patentinhaber: Solvay Fluor und Derivate GmbH, 30173 Hannover (DE)
(72) Erfinder: BROSCH, Carsten, D-30173 Hannover (DE); GRESS, Heinz, D-30457 Hannover (DE); RIELAND, Matthias, D-30539 Hannover (DE)
(74) Vertreter: Gosmann, Martin, Dr.
(86) Internationale Anmeldenummer: PCT/DE1999/002710
(87) Internationale Veröffentlichungsnummer: WO 2000/014040

(56) Entgegenhaltungen:
- EP-A- 0 370 688
- EP-A- 0 699 649
- EP-A- 0 905 085
- WO-A-90/08750
- DE-A- 2 310 749
- DATABASE WPI Section Ch, Week 199703 Derwent Publications Ltd., London, GB; Class E16, AN 1997-032536 XP002125734 & RU 2 058 283 C (KIROVO CHEPETSK CHEM COMBINE STOCK CO), 20. April 1996 (1996-04-20)

## Beschreibung

Die Erfindung bezieht sich auf ein Verfahren zur Herstellung von gereinigtem 1,1,1,3,3-Pentafluorbutan (HFC-365mfc).

1,1,1,3,3-Pentafluorbutan wird beispielsweise als Treibmittel für die Herstellung von geschäumten Kunststoffen verwendet. Es kann beispielsweise aus der entsprechenden Pentachlorbutanverbindung, Fluorwasserstoff und einem Fluorierungskatalysator hergestellt werden. Das auf diese Weise hergestellte 1,1,1,3,3-Pentafluorbutan kann Chlorwasserstoff, Fluorwasserstoff bzw. ungesättigte Kohlenstoffverbindungen enthalten, welche aus der Fluorierungsreaktion oder dem Einsatzmaterial entstammen. Es ist wünschenswert, aus dem verunreinigten Produkt ein gereinigtes Produkt herzustellen. Diese Aufgabe wird durch das Verfahren der vorliegenden Erfindung gelöst.

Das erfindungsgemäße Verfahren sieht vor, daß man zur Herstellung von gereinigtem 1,1,1,3,3-Pentafluorbutan mit einem verringerten Gehalt an HCl, HF und/oder an ungesättigten Verunreinigungen aus rohem 1,1,1,3,3-Pentafluorbutan, wobei man das rohe 1,1,1,3,3-Pentafluorbutan in der Flüssigphase mit zweiatomigen, an C-C-Mehrfachbindungen addierenden Molekülen behandelt und das behandelte 1,1,1,3,3-Pentafluorbutan abtrennt.

Druck und Temperatur werden so gewählt, daß man in der Flüssigphase arbeitet. Bevorzugt liegt der Druck bei 1 bis 5 atm (abs).

Mit den zweiatomigen Molekülen gelingt die Derivatisierung der ungesättigten Verbindungen zu nichttoxischen und/oder destillativ abtrennbaren Verbindungen.

Zur Verringerung des Gehaltes an ungesättigten Verbindungen setzt man als bevorzugtes zweiatomiges Molekül Chlorwasserstoff oder elementares Fluor, Chlor oder Wasserstoff ein.

Dabei kann man so vorgehen, daß man zunächst mittels der genannten zweiatomigen Moleküle den Gehalt an ungesättigten Verbindungen verringert und anschließend mittels eines festen anorganischen Sorptionsmittels weitere Verunreinigungen verringert.

Bevorzugte feste, anorganische Sorptionsmittel sind Aktivkohle und Sorbentien auf Basis von Aluminiumoxid oder Siliciumdioxid. Sie eignen sich besonders gut zur Abtrennung von Chlorwasserstoff und/oder Fluorwasserstoff.

Die Behandlung mit dem Sorptionsmittel wird vorteilhaft bei einer Temperatur von -30 °C bis +100 °C, vorzugsweise 15 bis 25 °C durchgeführt.

Besonders bevorzugt ist es, zur Verringerung des Gehaltes an ungesättigten Verbindungen elementares Fluor, vorzugsweise im Gemisch mit einem Inertgas wie Stickstoff oder Argon einzusetzen. Die Behandlung mit elementarem Fluor (bzw, seinen Gemischen mit Inertgas) führt man zweckmäßig bei einer Temperatur im Bereich von -80 bis +20 °C, vorzugsweise im Bereich von -20 bis -10 °C durch. Bereits bei niedriger Fluor-Konzentration, bis hin zu 10 Vol.-%, ist eine gute Wirkung zu beobachten.

Je nach Behandlungsdauer oder der eingesetzten Menge des die Reinigung bewirkenden Mittels kann man die Verunreinigungen mehr oder weniger vollständig abtrennen. So setzt man beispielsweise weniger Fluor ein, als für die Anlagerung an die ungesättigten Verunreinigungen benötigt wird, verbleibt eine entsprechende Menge der Verunreinigung im zu reinigenden Produkt. Dies kann aber durch einfache Handversuche und Produktanalyse ermittelt werden. Vorzugsweise führt man die Behandlung mit elementarem Fluor oder einem Gemisch von Fluor und Inertgas solange durch, bis ein 1,1,1,3,3-Pentafluorbutan erhalten wird, welches einen maximalen Gehalt an ungesättigten Chlor-Fluor-Verbindungen von 20 ppm und ungesättigten C2-Verbindungen von 10 ppm aufweist. Der Gehalt an Chlorwasserstoff und/oder Fluorwasserstoff wird solange durchgeführt, bis maximal je 1 ppm enthalten sind; hierzu verwendet man insbesondere die Behandlung mit amorphem Siliciumdioxid oder Aluminiumoxid.

Hat man die Behandlung des rohen Produktes mit einem zweiatomigen Molekül vorgenommen, kann die Auftrennung des resultierenden Produktes in reines Pentafluorbutan und andere Halogenkohlenwasserstoffe durch fraktionierte Kondensation oder beispielsweise auch durch Destillation erfolgen.

Mittels des erfindungsgemäßen Verfahrens ist es möglich, 1,1,1,3,3-Pentafluorbutan herzustellen, welches einen Gehalt von maximal 1 ppm an HF, 1 ppm an HCl, 10 ppm an ungesättigten (Chlor)-Fluor-Verbindungen und 10 ppm an ungesättigten C2-Verbindungen aufweist.

Das erfindungsgemäße Verfahren eignet sich hervorragend zur Reinigung von 1,1,1,3,3-Pentafluorbutan, welches durch Fluortrichlorethylen (als einziger Verunreinigung oder Bestandteil von Verunreinigungen) kontaminiert ist. Es wurde festgestellt, daß Fluortrichlorethylen besonders schwer abzutrennen ist, da es sehr unreaktiv ist. Mittels des erfindungsgemäßen Verfahrens kann selbst diese Verunreinigung auf einen Gehalt von maximal 20 ppm, ja auf einen Gehalt von weniger als 0,1 ppm verringert werden.

Aufgrund seiner Reaktionsträgheit kann Fluortrichlorethylen als Kontrollsubstanz bei der Überwachung ("beim Monitoring") der Reinigung von 1,1,1,3,3-Pentafluorbutan, welches durch Fluortrichlorethylen und weitere ungesättigte Verbindungen kontaminiert ist, verwendet werden. Es wurde überraschend gefunden, daß lediglich die Abreicherung von Fluortrichlorethylen überwacht werden muß und die gleichzeitige Überwachung der Abreicherung anderer ungesättigter Verbindungen entfallen kann. Wenn Fluortrichlorethylen auf den gewünschten Gehalt herabgedrückt ist, sind andere ungesättigte Verbindungen ebenfalls abgereichert. Dabei kann das Monitoring mittels GC-MS (SIM-Lauf = Selective Ion Mass) überwacht werden. Die Nachweisgrenze für CFC1111 bei der Bestimmung mittels Gaschromatographie (GC) - thermische Leitfähigsbestimmung (t.c.d., thermal conductivity detection) liegt bei 100 ppm. Mittels GC-MS (g.c. - m.s.) im SIM-Lauf-Modus (SIM run status) liegt die Nachweisgrenze bei 0,1 ppm CFC1111.

Mittels des erfindungsgemäßen Reinigungsverfahrens kann hochreines 1,1,1,3,3-Pentafluorbutan hergestellt werden. Bislang wurden derartige Reinigungsoperationen nur noch bei fluorierten oder vollhalogenierten Verbindungen hoher Stabilität durchgeführt. Insbesondere die Behandlung mit elementarem Fluor liefert ein überraschendes Resultat; denn man hätte statt der Anlagerung an ungesättigte Verbindungen die Substitutionsreaktion am Pentafluorbutan unter Bildung von Hexafluorbutan, Heptafluorbutan oder Perfluorbutan befürchten müssen. Die erfindungsgemäße Verwendung von Fluortrichlorethylen als Kontrollsubstanz beim Monitoring führt zu einer Zeitersparnis, da man sich bei der Aufnahme bzw. der Auswertung von Spektren auf den Bereich konzentrieren kann, in welchem Fluortrichlorethylen registriert wird. Das Arbeiten in der Flüssigphase spart Energie.

Die folgenden Beispiele sollen die Erfindung weiter erläutern, ohne sie in ihrem Umfang einzuschränken. Alle Versuche wurden in der Flüssigphase durchgeführt.

### Beispiel 1:

### Abtrennung ungesättigter Komponenten

a) Eingesetzt wurde HFC365mfc mit 0,22 % (Flächenprozent im Gaschromatogramm) CFC-1111 und 99,4 % HFC365mfc.
   aI) 84,8 g des Rohproduktes wurden mit 6,8 g Chlor in Gegenwart von 0,2 g FeCl₃ bei 40 °C zur Reaktion gebracht. Das Reaktionsgemisch wurde destilliert. Im Destillat waren noch 0,11 % CFC-1111 nachzuweisen. Die Menge an dieser Verunreinigung war somit halbiert worden.
   aII) 115,3 g des Rohproduktes wurden über einen Katalysator (4,5 Gew.-% Pd; 0,5 Gew.-% Rh, auf Aktivkohle) mit 0,8 g H₂ im Autoklaven hydriert. Im Reaktionsgemisch waren nur noch 0,03 % CFC-1111 enthalten.
b) Eingesetzt wurde HFC365mfc als Rohprodukt mit 0,16 % (Flächenprozent im Gaschromatogramm) CFC-1111 und 99,68 % HFC365mfc.
   bI) 93,6 g des Rohproduktes wurden mit 2,3 g Jod (in Form von KJ/J₂ umgesetzt. Im Reaktionsgemisch waren noch 0,08 % CFC-1111 enthalten.
   bII) 195,6 g des Rohproduktes wurden mit 0,72 Litern eines F₂/N₂-Gemisches (10 % F₂) bei -20 °C umgesetzt. Im Reaktionsgemisch war kein CFC-1111 mehr nachweisbar.

### Beispiel 2:

### Abtrennung von CFC-1111 und sauren Komponenten

Eingesetzt wurden 272 kg HFC365mfc mit einem Gehalt an CFC-1111 von ca. 0,2 % (Flächenprozent im GB). Das entspricht ca. 2.000 ppm. Die Reaktion wurde in einem Reaktor mit Umwälzkreislauf durchgeführt. Bei Kühlung auf -12 °C wurden pro Stunde 150 l eines F₂/N₂-Gemisches durchgeleitet (3 Vol.-% F₂). Dauer: 12 Stunden. Danach wurde das Reaktionsgemisch erwärmt und HFC365mfc abdestilliert. Der Gehalt von sauren Bestandteilen (insbesondere HCl und HF) wurde das Destillat mit einem Adsorbens auf SiO₂-Basis kontaktiert. Hierzu wurde als Adsorbens "AF400®" verwendet. Dies ist ein aluminiumfreies, perlförmiges Adsorbens auf SiO₂-Basis mit einem Porendurchmesser von 400 Å (40 nm), Lieferant: Kali-Chemie/Engelhard, Nienburg.

Nach Abtrennen des HFC365mfc vom Adsorbens lag der Gehalt an HCl unterhalb von 1 ppm, der Gehalt an HF ebenfalls unterhalb von 1 ppm.

(Anmerkung: Zur Bestimmung des Gehaltes an CFC-1111 im Produkt wurde der Sim-MS-Status des GC-MS-Gerätes gefahren. Vorab wurde eine Eichkurve erstellt, um den Ionenstrom bei 0,1 ppm CFC-1111 und 10 ppm zu bestimmen (mittels hierfür angefertigter Mischungen von HFC365mfc und CFC-1111 der entsprechenden Konzentration). Mittels der Eichkurve wurden dann die beobachteten Ionenströme und die zugehörigen Konzentrationen an CFC-1111 korreliert.

## Patentansprüche

1. Verfahren zur Herstellung von gereinigtem 1,1,1,3,3-Pentafluorbutan mit einem verringerten Gehalt an HCl, HF und/oder an ungesättigten Verunreinigungen aus rohem 1,1,1,3,3-Pentafluorbutan, wobei man das rohe 1,1,1,3,3-Pentafluorbutan in der Flüssigphase mit zweiatomigen, an C-C-Mehrfachbindungen addierenden Molekülen behandelt und das behandelte 1,1,1,3,3-Pentafluorbutan abtrennt.

2. Verfahren nach Anspruch 1, **dadurch gekennzeichnet, dass** man zusätzlich ein festes, anorganisches Sorptionsmittel einsetzt.

3. Verfahren nach Anspruch 2, **dadurch gekennzeichnet, dass** man das Sorptionsmittel zur Abtrennung von HCl und/oder HF einsetzt.

4. Verfahren nach Anspruch 2, **dadurch gekennzeichnet, dass** man die Behandlung mit dem Sorptionsmittel bei einer Temperatur von -30 bis +100, vorzugsweise 15 bis 25 °C durchführt.

5. Verfahren nach Anspruch 1, **dadurch gekennzeichnet, dass** man zur Verringerung des Gehaltes an ungesättigten Verbindungen als zweiatomiges Molekül HCl oder elementares Fluor, Chlor oder Wasserstoff einsetzt.

6. Verfahren nach Anspruch 5, **dadurch gekennzeichnet, dass** man elementares Fluor im Gemisch mit einem Inertgas einsetzt.

7. Verfahren nach Anspruch 6, **dadurch gekennzeichnet, dass** man die Behandlung mit elementarem Fluor bei einer Temperatur im Bereich von -80 bis +20 °C durchführt.

8. Verfahren nach Anspruch 6 oder 7, **dadurch gekennzeichnet, dass** man die Behandlung mit elementarem Fluor oder einem Gemisch von Fluor und Inertgas solange durchführt, bis ein 1,1,1,3,3-Pentafluorbutan erhalten wird, welches einen maximalen Gehalt an ungesättigten (Chlor)-Fluor-Verbindungen von 20 ppm und ungesättigten C2-Verbindungen von 10 ppm aufweist.

9. Verfahren nach Anspruch 2, **dadurch gekennzeichnet, dass** man die Behandlung mit amorphen SiO₂ oder Al₂O₃ solange durchführt, bis der Gehalt an HCl und/oder HF maximal je 1 ppm beträgt.

## Claims

1. A process for producing purified 1,1,1,3,3-pentafluorobutane with a decreased content of HCl, HF and/or unsaturated impurities from crude 1,1,1,3,3-pentafluorobutane, whereby the crude 1,1,1,3,3-pentafluorobutane is treated in the liquid phase with diatomic molecules which add to C-C-multiple bonds, and separating the treated 1,1,1,3,3-pentafluorobutane.

2. A process according to claim 1, **characterized in** the additional use of a solid, inorganic sorption agent.

3. A process according to claim 2, **characterized in that** the sorption agent is used to separate HCl and/or HF.

4. A process according to claim 2, **characterized in that** the treatment with the sorption agent is carried out at a temperature in the range from -30 to +100 °C, preferably in the range from 15 to 25 °C.

5. A process according to claim 1, **characterized in that** as diatomic molecule, HCl elemental fluorine, chlorine or H₂ is used to decrease the unsaturated compound content.

6. A process according to claim 5, **characterized in that** elemental fluorine is used in admixture with an inert gas.

7. A process according to claim 6, **characterized in** the treatment with elemental fluorine at a temperature in the range from -80 to +20 °C.

8. A process according to claim 6 or 7, **characterized in that** the treatment with elemental fluorine or a with a mixture of fluorine and an inert gas is carried out until a 1,1,1,3,3-pentafluorobutane product is obtained containing not more than 20 ppm unsaturated (chlorine)-fluorine compounds and not more than 10 ppm unsaturated C2-compounds.

9. A process according to claim 2, **characterized in that** the treatment with amorphous SiO₂ or Al₂O₃ is carried out until a content of maximum 1 ppm of HCl and/or HF is obtained.

## Revendications

1. Procédé de fabrication de 1,1,1,3,3-pentafluorobutane purifié présentant une teneur réduite en HCl, HF et/ou en impuretés insaturées à partir du 1,1,1,3,3-pentafluorobutane brut, dans lequel on traite le 1,1,1,3,3-pentafluorobutane brut en phase liquide avec des molécules biatomiques s'additionnant à des liaisons multiples C-C, et on sépare le 1,1,1,3,3-pentafluorobutane traité.

2. Procédé selon la revendication 1, **caractérisé en ce que** l'on utilise en outre un sorbant solide inorganique.

3. Procédé selon la revendication 2, **caractérisé en ce que** l'on utilise le sorbant pour séparer HCl et/ou HF.

4. Procédé selon la revendication 2, **caractérisé en ce que** l'on réalise le traitement avec le sorbant à une température comprise dans la gamme de -30 à + 100, de préférence de 15 à 25 °C.

5. Procédé selon la revendication 1, **caractérisé en ce que**, pour réduire la teneur en composés insaturés, on utilise comme molécule biatomique HCl ou du fluor, du chlore ou de l'hydrogène élémentaire.

6. Procédé selon la revendication 5, **caractérisé en ce que** l'on utilise du fluor élémentaire en mélange avec un gaz inerte.

7. Procédé selon la revendication 6, **caractérisé en ce que** l'on réalise le traitement avec du fluor élémentaire à une température comprise dans la gamme de - 80 °C à + 20 °C.

8. Procédé selon la revendication 6 ou 7, **caractérisé en ce que** l'on réalise le traitement avec du fluor élémentaire ou un mélange de fluor et de gaz inerte jusqu'à ce que l'on obtienne un 1,1,1,3,3-pentafluorobutane qui présente une teneur maximum en composés (chloro)-fluorés insaturés de 20 ppm et en composés C2 insaturés de 10 ppm.

9. Procédé selon la revendication 2, **caractérisé en ce que** l'on réalise le traitement avec SiO₂ ou Al₂O₃ amorphe jusqu'à ce que la teneur en HCl et/ou HF soit au maximum respectivement de 1 ppm.
